Europäisches Patentamt

European Patent Office

Office européen des brevets

(19)

(11) Numéro de publication: **0 144 272**
**A1**

(12) ## DEMANDE DE BREVET EUROPEEN

(21) Numéro de dépôt: **84402484.4**

(22) Date de dépôt: **04.12.84**

(51) Int. Cl.⁴: **H 01 J 35/30**
**H 01 J 35/14, A 61 B 6/02**

(30) Priorité: **06.12.83 FR 8319484**

(43) Date de publication de la demande:
**12.06.85 Bulletin 85/24**

(84) Etats contractants désignés:
**DE GB NL**

(71) Demandeur: **THOMSON-CGR**
**13, square Max-Hymans**
**F-75015 Paris(FR)**

(72) Inventeur: **Plessis, André**
**Thomson-CSF SCPI 173, bld. Haussmann**
**F-75379 Paris Cedex 08(FR)**

(72) Inventeur: **Le Guen, Jacques**
**Thomson-CSF SCPI 173, bld. Haussmann**
**F-75379 Paris Cedex 08(FR)**

(74) Mandataire: **Barbin le Bourhis, Joel et al,**
**THOMSON-CSF SCPI 173, boulevard Haussmann**
**F-75379 Paris Cedex 08(FR)**

(54) **Installation de radiodiagnostic pour examens stéréoscopiques ou conventionnels.**

(57) L'invention concerne une installation de radiodiagnostic pour examens stéréoscopiques ou conventionnels, permettant notamment des cadences rapides de prises de clichés stéréographiques, avec une base stéréo ajustable.

L'installation (1) comporte un tube radiogène (2) et des moyens de déviation (10) d'un faisceau d'électrons (7). Elle comporte en outre des moyens de commande de déviation (13) agissant sur les moyens de déviation (10) en synchronisme avec un générateur haute tension (15) et un récepteur d'images (3), pour déterminer des déviations successives du faisceau d'électrons (7).

FIG_1

# INSTALLATION DE RADIODIAGNOSTIC POUR
# EXAMENS STEREOSCOPIQUES OU CONVENTIONNELS

L'invention concerne une installation de radiodiagnostic pour examens stéréoscopiques ou conventionnels, destinée notamment à la prise de clichés stéréographiques à cadence rapide.

L'effet stéréographique est obtenu en radiographiant un même sujet à partir de deux sources, localisées indépendamment dans l'espace, déterminant chacune une image ou cliché du sujet vu sous des incidences différentes ; ces deux images constituant un couple de clichés stéréographiques. Une vision stéréoscopique du sujet est obtenue en regardant ces deux images en vision binoculaire, c'est-à-dire que chacune des images est regardée indépendamment par chacun des deux yeux.

Des installations stéréographiques ont été réalisées qui, en ce qui concerne cette localisation indépendante des sources de rayonnement X servant à la prise de chacun des clichés, correspondent à l'un des modèles suivants :

- installation comprenant des tubes radiogènes destinés chacun à la prise d'un cliché. L'inconvénient de cette solution, outre son coût, est que la distance minimum entre sources ou foyers reste grande, du fait de l'encombrement des tubes radiogènes ; aussi dans ce cas, la distance entre sources ou foyers, appelée base stéréo, n'est pas compatible avec des agrandissements moyens ou forts;

- installation à unique tube radiogène déplaçable entre deux clichés. Un des inconvénients de ce système est qu'il exige, pour le déplacement du tube radiogène, des moyens mécaniques qui limitent considérablement la fréquence de prise de clichés;

- installation comportant un tube spécialisé à deux foyers décalés, chacun des faisceaux issus des deux foyers étant destiné à une prise de cliché. L'inconvénient dans ce dernier cas est que la base stéréo est fixe. Il est à remarquer également que dans ce cas,

2

l'utilisation du tube radiogène pour des clichés conventionnels, c'est-à-dire non stéréo, présente l'inconvénient de produire des clichés excentrés ; la source du faisceau X utilisée étant elle-même excentrée par rapport à une fenêtre de sortie et des moyens de collimation de ce faisceau.

Un but de la présente invention est de permettre, avec une installation de radiodiagnostic munie d'un unique tube radiogène, des prises de clichés stéréographiques à une cadence rapide, avec une base stéréo continuement variable et ajustable en fonction de l'agrandissement désiré, et enfin, de permettre à l'installation selon l'invention la prise de clichés conventionnels dans les conditions optimums.

Selon l'invention, une installations de radiodiagnostic pour examens stéréoscopiques ou conventionnels, comportant un tube radiogène produisant un rayonnement X dont le foyer peut occuper au moins deux positions fixes dans l'espace, ledit tube comportant une anode et une cathode génératrice d'un faisceau d'électrons bombardant l'anode en un point où est formé ledit foyer, ladite installation comportant un récepteur d'images et un générateur haute tension mutuellement synchronisés grâce à des moyens de synchronisation, est caractérisée en ce qu'elle comporte en outre des moyens de déviation, actionnés par des moyens de commande de déviation coopérant avec le récepteur d'images et le générateur haute tension pour réaliser une déviation du faisceau d'électrons permettant d'obtenir lesdites positions du foyer.

L'invention sera mieux comprise grâce à la description qui suit et aux deux figures annexées parmi lesquelles :

- la figure 1 : montre schématiquement l'organisation de moyens caractéristiques de l'installation selon l'invention,

- la figure 2 : montre, par un diagramme temporel, le fonctionnement de l'installation conforme à l'invention.

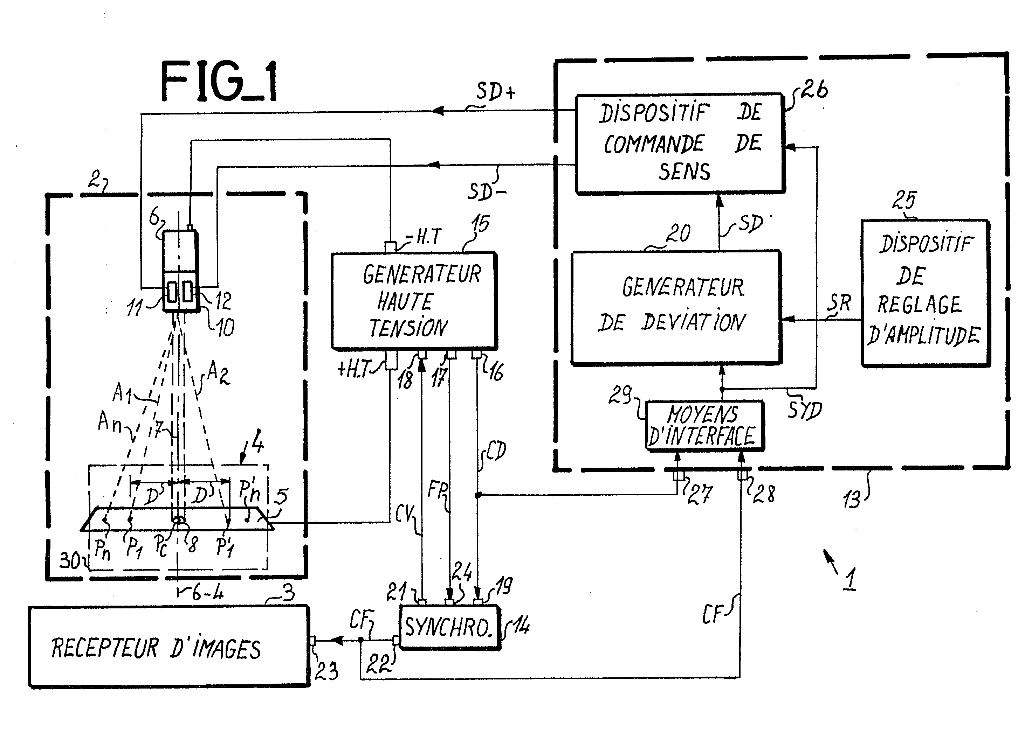

La figure 1 montre schématiquement des moyens caractéristiques d'une installation 1 de radiodiagnostic selon l'invention, ces moyens étant représentés sous la forme de blocs fonctionnels organisés autour d'un tube radiogène 2 et comportant notamment un récepteur d'images 3 synchronisé avec un générateur de haute tension 15 ; la disposition du tube radiogène 2 et du récepteur d'images 3 par rapport à un sujet (non représenté) à examiner étant connue, elle n'est pas montrée, de même que d'autres moyens connus nécessaires au fonctionnement mais non utiles à la compréhension de l'invention.

Le tube radiogène 2, est muni d'une anode 4 d'un type classique, dont la surface visible dans le plan de la figure constitue une cible anodique 5 ; cette anode pouvant être soit une anode fixe soit une anode tournante. Le tube radiogène 2 comporte également une cathode 6, génératrice d'électrons d'une manière connue (non représentée), comme par thermo-émission par exemple ; cette cathode 6 est disposée de manière classique, son axe longitudinal constituant un axe cathode-anode 6-4 passant par la cible anodique 5, laquelle cible 5 est bombardée par un faisceau d'électrons 7 issu de la cathode 6.

Ainsi qu'il est davantage expliqué dans une suite de la description, l'axe cathode-anode 6-4 représente un axe de repos du faisceau d'électrons 7 dont l'impact, sur une position centrale PC de la cible anodique 5, détermine un foyer 8 ; ce foyer constituant la source d'un rayonnement X (non représentée).

Afin d'obtenir, sur la cible anodique 5, des positions fixes différentes P1, ..., Pn, de la formation du foyer 8, il est procédé dans l'invention à une déviation du faisceau d'électrons 7.

A cette fin, il est utilisé dans l'exemple non limitatif décrit, un dispositif de déviation 10 centré autour de l'axe de repos 6-4 et, disposé dans le prolongement de la cathode 4 entre cette dernière et l'anode 6. Le dispositif de déviation 10 peut être : soit du type électromagnétique, muni par exemple de bobines magnétiques (non représentées), auquel cas il peut être extérieur au tube radiogène 2 ; soit du type électrostatique ainsi que dans l'exemple décrit, où il est muni de plaques déviatrices 11, 12 électrostatiques.

4

Ces plaques déviatrices 11, 12 sont actionnées par des moyens de commande de déviation 13 montrés dans un cadre en traits pointillés, en synchronisme avec des commandes et des informations échangées d'une manière classique entre le récepteur d'images 3 et le générateur de haute tension 15.

Il est connu que dans une installation de radiodiagnostic, le générateur haute tension, bien que sommairement représenté sur la figure 1, constitue un ensemble relativement complexe, comportant notamment ou étroitement associé avec un pupitre de commande ; cette partie étant bien connue de l'homme de métier, elle n'est pas représentée, le pupitre de commande étant dans l'exemple décrit englobé dans le générateur haute tension. C'est généralement au niveau du pupitre de commande qu'un opérateur détermine, en radiographie par exemple, les caractéristiques d'une suite de séquences, en fonction de la nature des clichés à obtenir et des possibilités qu'offre l'installation.

L'opérateur doit ainsi tenir compte des caractéristiques techniques du récepteur d'images, pour déterminer par exemple, l'intensité et la durée du rayonnement X (temps de pose). Le récepteur d'images peut être constitué par exemple, par une chaîne vidéo à mémoire dont la structure est classique, ou ainsi que dans l'exemple non limitatif décrit par un changeur de film, classique également.

Généralement, les changeurs de film comportent, d'une manière connue, un magasin où des films vierges (non représentés) prédécoupés par exemple, sont stockés à l'abri du rayonnement X. Pour la prise d'un cliché, un film est déplacé, dans une opération appelée chargement de film, vers une chambre d'exposition dans laquelle ce film est impressionné par le rayonnement X ; il est ensuite, dans une opération inverse appelée déchargement de film, ramené à l'abri du rayonnement X, lequel rayonnement X doit être interrompu durant les déplacements du film. Ces opérations s'effectuent en liaison avec le générateur haute tension, par l'intermédiaire de moyens d'interface dont est muni le changeur de film. Ces moyens d'interface ont la connaissance des caractéristiques du

5

changeur de film, et constituent un dispositif de synchronisation entre les parties mécaniques de ce changeur et le générateur haute tension.

Ainsi, par exemple, le dispositif de synchronisation ayant reçu du générateur haute tension une demande de début de séquence, il transmet un ordre de chargement de film aux moyens mécaniques du changeur, et transmet en retour au générateur haute tension, quant cette fonction est accomplie, une information dite validation de chargement. Cette information de validation de chargement sert dans le générateur haute tension à commander l'application de la haute tension au tube radiogène, pour générer le rayonnement X durant un temps appelé temps de pose, ces opérations s'effectuant en sens inverse à partir de la fin de ce temps de pose.

Ces échanges classiques entre le générateur haute tension 15 et le récepteur d'images 3 sont utilisés dans la présente invention, pour permettre en plus d'un fonctionnement conventionnel, un fonctionnement en mode stéréographique. Pour la prise de clichés stéréographiques, les opérations qui viennent d'être ci-dessus mentionnées sont exécutées deux fois consécutivement, avec un même temps de pose mais avec un rayonnement X émis sous une incidence différente d'une fois à l'autre. Cette dernière condition est remplie dans l'installation 1 conforme à l'invention, grâce aux moyens de commande de déviation 13 qui, d'une part, coopèrent avec le dispositif de déviation 10 pour provoquer une déviation du faisceau d'électrons 7, et d'autre part, sont commandés a partir des informations échangées entre le générateur haute tension 15 et le changeur de film ou récepteur d'images 3 ; les moyens de synchronisation précédemment cités que comporte ce dernier sont, dans l'exemple non limitatif décrit, pour une meilleure clarté de la description, représentés séparés du récepteur d'images 3 et affectés d'un repère 14.

La description qui suit montre à titre d'exemple non limitatif, des connexions établies entre le générateur haute tension 15, le récepteur d'images 3, le tube radiogène 2 et les moyens de commande de déviation 13, ces connexions montrées étant limitées à celles nécessaires à comprendre l'invention.

Le générateur haute tension 15 délivre la haute tension au tube radiogène 2, par une première et une seconde borne de sortie +HT, -HT, respectivement reliées à l'anode 4 et à la cathode 6. Le générateur haute tension comporte en outre une troisième et une quatrième sortie 16, 17, et une première entrée 18 :

- la troisième sortie 16, reliée à une seconde entrée 19 des moyens de synchronisation 14, délivre un signal de début de séquence CD ; ce signal pouvant comporter en outre des informations sur la nature de la séquence, telles que par exemple, radiographie en mode conventionnel ou en mode stéréographique ;

- la quatrième sortie 17, reliée à une troisième entrée 24 des moyens de synchronisation 14, délivre une information FP de fin de pose ;

- la première entrée 18 est reliée à une cinquième sortie 21 des moyens de synchronisation 14, par laquelle ceux-ci délivrent une information de validation de chargement VC.

Les moyens de synchronisation 14 sont reliés par une sixième sortie 22 à une quatrième entrée 23 du récepteur d'images 3 ; cette liaison servant à transmettre au récepteur d'images 3 une commande de chargement ou de déchargement de film CF.

Les moyens de commande de déviation 13 sont chargés d'assurer, par des moyens traditionnels non représentés, les fonctions suivantes :

a) génération, quand le fonctionnement s'effectue selon le mode stéréographique, d'un signal de déviation SD fourni par un générateur de déviation 20. Ce générateur de déviation pouvant comporter par exemple à cet effet, un amplificateur générant ce signal SD, suivi d'un moyen de commutation autorisant la sortie du signal SD, sous le contrôle d'un signal de synchronisation de déviation SYD. A cet effet, la commande de début de séquence CD délivrée par le générateur de haute tension 15 et, la commande de chargement de film CF délivrée par les moyens de synchronisation 14, sont également fournies, par l'intermédiaire respectivement d'une cinquième et sixième entrée 27 et 28, aux moyens de commande de déviation 13 où ils sont appliqués à un dispositif d'interface 29 ; ce dispositif d'interface permettant, si nécessaire, d'adapter les caractéristiques de ces signaux aux caractéristiques d'entrée des moyens de commande de déviation 13. Le dispositif d'interface 29 comporte en outre des moyens classiques, destinés à autoriser le passage de la commande de chargement de film CF si, le signal de début de séquence indique un fonctionnement en mode stéréographique ; dans ce cas, la commande de chargement de film CF constitue un signal de synchronisation de déviation SYD appliqué au générateur de déviation 20, pour autoriser la sortie du signal de déviation SD. Dans le cas de l'exemple non limitatif décrit, où le dispositif de déviation 10 est du type électrostatique, le signal de déviation SD peut consister en une tension continue, le niveau de cette tension déterminant l'amplitude de la déviation du faisceau d'électrons 7.

b) réglage de l'amplitude de la déviation, assuré par un dispositif de réglage d'amplitude 25 comportant à cet effet, d'une manière classique, des moyens numériques ou analogiques, tels que par exemple un potentiomètre combiné à une source de tension continue. Il est ainsi généré une tension de référence SR, appliquée au générateur de déviation 20, et dont le réglage par le potentiomètre détermine l'amplitude du signal de déviation SD.

c) détermination d'une première ou d'une seconde commande de déviation, de sens opposés. Cette fonction est assurée par un dispositif de commande de sens 26, auquel est appliqué le signal de déviation SD fourni par le générateur de déviation 20. Ce signal SD peut être traité de différentes manières toutes connues en elles-mêmes, permettant d'obtenir un premier et un second signal de commande de déviation SC+, SC-, correspondant chacun à un sens opposé de la déviation.

Dans ce dispositif de commande de sens 26, le signal de déviation SD fourni par le générateur de déviation 20, peut être appliqué par exemple, simultanément à deux moyens amplificateurs ayant un même coefficient d'amplification et, actionné selon des

8

sens opposés ; ils fournissent ainsi, l'un le premier signal de commande de déviation SD+, l'autre le second signal de commande SD-, ces signaux ayant une même amplitude mais une polarité opposée, par rapport à un niveau de référence (non représenté) relié par exemple au -HT. Afin de permettre uniquement la sortie de l'un ou l'autre des signaux de commande de déviation SD+, SD-, les sorties des moyens amplificateurs sont reliés à un moyen de commutation qui, selon qu'il est actionné ou non, autorise le passage de l'un ou l'autre de ces signaux SD+, SD-. L'actionnement de ce second moyen de commutation peut être obtenu par exemple, à partir d'une bascule à deux positions dont la première position correspond à autoriser la sortie du premier signal de commande SD+, et la seconde position à autoriser la sortie du second signal de commande SD-; cette bascule pouvant être elle-même actionnée par le signal de synchronisation de déviation SYD, qui pour cela, est également appliqué au dispositif de commande de sens 26.

Il est ainsi possible d'obtenir une déviation du faisceau d'électrons en synchronisme avec les chargements de films opérés, en commandant l'action des moyens de commande de déviation 13 par les signaux CD, CF, servant de manière classique à la synchronisation mutuelle du récepteur d'images 3 et du générateur haute tension 15 ; les moyens de commande de déviation 13 étant, à cette fin, reliés aux moyens de synchronisation 14, à la seconde entrée 19 et à la sixième sortie 22 de ces derniers.

Cette déviation du faisceau d'électrons peut lui conférer n directions moyennes A1, ..., An, de part et d'autre de l'axe anode-cathode 6-4, cet axe anode-cathode constituant un axe de repos en l'absence de commande de déviation SD+, SD-.

Comme il a été précédemment expliqué, l'impact du faisceau d'électrons 7 engendre un foyer 8 d'où est émis un rayonnement X (non représenté); en l'absence de signaux de commande de déviation SD+, SD-, le foyer 8 occupe une position centrale PC sur la cible anodique 5. Cette position centrale du foyer 8 correspond à l'axe d'une fenêtre de sortie du rayonnement X disposée parallèlement au plan de la figure, et permet la prise de clichés radiographiques (non stéréo), dans les mêmes conditions qu'une installation de radiodiagnostic conventionnelle ; cette fenêtre de sortie étant sur la figure 1 symbolisée par un cadre en traits pointillés et repérée 30.

Avec la déviation du faisceau, le foyer peut être formé sur la cible anodique à n et n' positions P1, ..., Pn, et P1', ..., Pn', respectivement de part et d'autre de la position centrale PC. Ainsi pour la stéréographie, avec la déviation du faisceau d'électrons, n bases stéréo peuvent être obtenues, constituées chacune par deux positions fixes successives P1, P1' à Pn, Pn' occupées par le foyer en fonction de l'amplitude des signaux de déviation SD=, SD- ; ces deux positions successives étant de préférence à une même distance D de la position centrale PC, comme dans l'exemple de la figure 1 où des premières positions P1, P1' disposées respectivement de part et d'autre de la position centrale PC, constituent une première base stéréo.

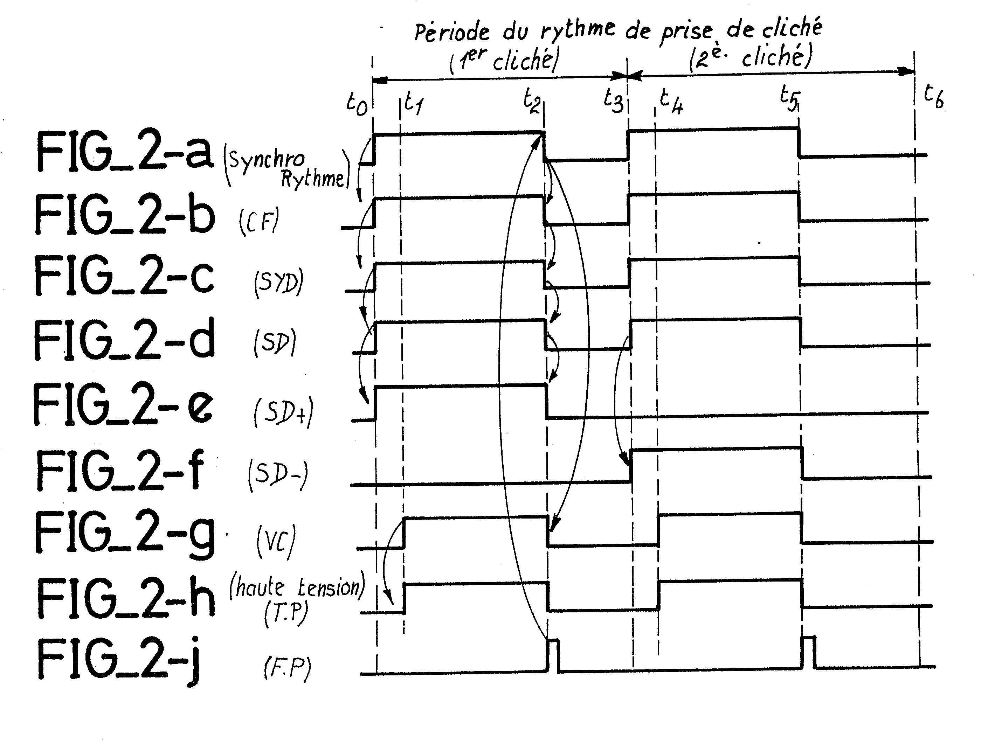

La figure 2 montre par un diagramme temporel, le fonctionnement en stéréographie, de l'installation 1 conforme à l'invention. Ce diagramme illustre une suite de fonctions débutée à partir d'une commande de début de séquence CD (non représentée) et accomplie pour la prise de deux clichés successifs ; chaque prise de cliché correspondant à une période d'un rythme de cliché, établie en coopération avec le générateur haute tension 15, au niveau des moyens de synchronisation 14 par une fonction appelée synchro rythme :

- on trouve à l'instant t0 : ligne A, début de la période du rythme de prise de cliché (synchro rythme) ; ligne B, début de la commande de chargement de film CF fournie par les moyens de synchronisation 14 au récepteur d'images 3, et aux moyens de commande de déviation 13 ; ligne C, début du signal de synchronisation de déviation SYD généré par le dispositif d'interface 29 ; ligne D, début du signal de déviation SD fourni par le générateur de déviation 20 ; ligne E, début de la première commande de déviation SD+ fournie par le générateur de commande de sens 26 à la première plaque déviatrice 11 ; il est à noter que l'amplitude (non représentée) du signal de déviation SD, a pu être préalablement réglée grâce au dispositif de réglage d'amplitude 25, pour correspondre à l'une des positions P1, ..., Pn et P1', ... Pn' successivement désirées. Les différences de temps d'établissement entre ces fonctions étant très faibles par rapport à la période, elles ne sont pas représentées, mais l'enchaînement de ces fonctions est illustré sur la figure 2 grâce à des flèches partant de l'instant t0 : il est ainsi montré que l'établissement de la période (synchro rythme) entraîne la commande de chargement de film CF, laquelle entraîne à son tour l'établissement du signal de synchronisation de déviation SYD, à partir duquel est établi le signal de déviation SD qui à son tour provoque l'établissement de la première commande de déviation SD+.

On trouve à l'instant t1 (le temps entre l'instant t0 et l'instant t1 correspondant au temps nécessaire au chargement du film) : ligne G, début du signal de validation VC de chargement du film, transmis par des moyens de synchronisation 14 au générateur haute tension 15 ; ligne H, début de l'application de la haute tension. La flèche partant de l'instant t1, montre que c'est l'établissement du signal de validation VC qui provoque l'établissement de la haute tension appliquée au tube radiogène 2. Il est à noter que le faisceau d'électrons 7 est alors établi et que le foyer 8 est formé, à la position P1 par exemple.

On trouve à l'instant t2 : ligne H, fin de l'application de la haute tension ; ligne J, apparition du signal de fin de pose FP généré avec la fin de l'application de la haute tension ; ligne A, fin d'une première partie de la période de prise de cliché (synchro rythme) ; ligne B, fin de la commande chargement de film CF, cette fin de commande constituant un ordre de déchargement du film exposé ; ligne G, fin du signal de validation de chargement VC ; ligne C, fin du signal de synchronisation SYD ; ligne D, fin du signal de déviation SD ; ligne E, fin de la première commande de déviation SD+. Les flèches partant de l'instant t2 montrent : qu'à la fin de l'application de la haute tension est généré le signal de fin de pose FP, lequel provoque la fin de la première partie de la période de prise de cliché (synchro rythme), que la fin de cette première partie de période commande simultanément la fin de la commande de chargement de film CF et la fin du signal de validation de chargement VC, et que la fin de la commande chargement de film CF détermine la fin du signal de synchronisation de déviation SYD, lequel entraîne dans un même ordre que celui montré à l'instant t0, la fin du signal de déviation SD et de la première commande de déviation SD+ ; le faisceau d'électrons 7 étant coupé à la fin de l'application de la haute tension.

L'instant t3 correspond à la fin de la période de prise du premier cliché et au début d'une seconde période de prise d'un second cliché.

On trouve à l'instant t3 : ligne A, début de la seconde période du rythme de prise de cliché (synchro rythme) ; ligne B, début de la commande de chargement de film CF ; ligne C, début du signal de synchronisation de déviation SYD ; ligne D, début du signal de déviation SD ; ligne E, début de la seconde commande de déviation SD-, fournie par le générateur de commande de sens 26, et appliquée à la seconde plaque déviatrice 12 ; cette succession de fonctions s'établit d'une même manière que celle montrée a l'instant t0, sauf à partir de l'établissement du signal de déviation SD qui, dans cette seconde prise de cliché, détermine la seconde commande de déviation SD-.

On trouve à l'instant t4 : ligne G, début du signal de validation VC de chargement de film ; ligne H, début de l'application de la haute tension. Le faisceau d'électrons 7 est alors à nouveau établi, le foyer 8 étant généré cette fois à la position P1'.

On trouve à l'instant t5 : ligne H, fin de l'application de la haute tension ; ligne J, apparition du signal de fin de pose FP ; ligne A, fin de la première partie de la seconde période de prise de cliché (cette première partie représentant l'exposition d'un film) ; ligne B, fin de la commande de chargement de film ; ligne C, fin du signal de synchronisation de déviation SYD ; ligne D, fin du signal de dévia-

12

tion SD ; ligne F, fin de la seconde commande de déviation SD- ; ligne G, fin du signal de validation de chargement VC.

L'instant t6 représente la fin de la seconde période du rythme de prise de cliché, relative au second cliché.

Cette suite de fonctions a permis, selon un fonctionnement automatique, la prise de deux clichés stéréographiques durant des temps de pose identiques, représentés ligne H par les applications de haute tension ; la différence étant que, durant chacun de ces temps de pose, la commande de déviation SD+, SD- appliquée aux dispositifs de déviation 10 est différente, de manière à engendrer successivement au foyer 8 deux positions P1, P1' différentes.

Il est possible, selon le type de récepteur d'images employé, ou selon le type d'une autre configuration d'une installation déjà existante, d'organiser différemment le fonctionnement d'une installation 1 de radiodiagnostic selon l'invention. L'important est de déterminer avec un unique tube radiogène 2, comme il a été montré dans cette description, un rayonnement X dont le foyer 8 occupe dans l'espace une position P1, P1' différente d'un cliché à l'autre, grâce à une déviation d'amplitude réglable du faisceau d'électrons 7 tout en respectant les conditions optimums nécessaires à une radiographie conventionnelle.

Un avantage important de l'invention est qu'elle est facilement applicable à des installations de radiographie conventionnelle existantes, que l'on désire adapter en outre à des examens stéréoscopiques.

0144272

13

REVENDICATIONS

1. Installation de radiodiagnostic pour examens stéréoscopiques ou conventionnels, comportant un tube radiogène (2) produisant un rayonnement X dont le foyer (8) peut occuper au moins deux positions (P1, P1') fixes dans l'espace, ledit tube (2) comportant une anode (4) et, une cathode (6) génératrice d'un faisceau d'électrons (7) bombardant l'anode (4) en un point où est formé ledit foyer (8), ladite installation (1) comportant un récepteur d'images (3) et un générateur haute tension (15) mutuellement synchronisés grâce à des moyens de synchronisation (14), caractérisée en ce qu'elle comporte en outre des moyens de déviation (10), actionnés par des moyens de commande de déviation (13) coopérant avec le récepteur d'images (3) et le générateur haute tension (15) pour réaliser une déviation du faisceau d'électrons (7) permettant d'obtenir lesdites positions (P1, P1') du foyer (8).

2. Installation de radiodiagnostic selon la revendication 1, caractérisée en ce que les moyens de déviation (10) sont du type électrostatique.

3. Installation de radiodiagnostic selon la revendication 1, caractérisée ne ce que les moyens de déviation (10) sont du type électromagnétique.

4. Installation de radiodiagnostic selon l'une des revendications précédentes, caractérisée en ce que les moyens de déviation (10) sont contenus dans le tube radiogène (2).

5. Installation de radiodiagnostic selon la revendication 1, caractérisée en ce que les moyens de commande de déviation (13) sont reliés aux moyens de synchronisation (14), de manière à utiliser des signaux (CD, CF) servant à la synchronisation mutuelle du générateur haute tension (15) et du récepteur d'images (3).

6. Installation de radiodiagnostic selon l'une des revendications précédentes, caractérisée en ce que les moyens de commande de déviation (13) comportent un dispositif de réglage d'amplitude (25) de la déviation.

14

7. Installation de radiodiagnostic selon l'une des revendications précédentes, caractérisée en ce que les moyens de commande de déviation (13) comportent un dispositif de commande de sens (26) de la déviation.

8. Installation de radiodiagnostic selon la revendication 1, caractérisée en ce qu'elle comporte en outre une fenêtre de sortie (30) du rayonnement X, et en ce que le foyer (8) est formé à une position centrale (PC), centrée par rapport à la fenêtre de sortie (30).

**FIG_1**

GENERATEUR HAUTE TENSION (15)

DISPOSITIF DE COMMANDE DE SENS (26)

GENERATEUR DE DEVIATION (20)

DISPOSITIF DE REGLAGE D'AMPLITUDE (25)

MOYENS D'INTERFACE (29)

RECEPTEUR D'IMAGES (3)

SYNCHRO. (14)

1/2

0144272

Période du rythme de prise de cliché
(1$^{er}$ cliché)          (2$^{è}$ cliché)

$t_0$  $t_1$        $t_2$    $t_3$  $t_4$        $t_5$        $t_6$

FIG_2-a (Synchro Rythme)

FIG_2-b (CF)

FIG_2-c (SYD)

FIG_2-d (SD)

FIG_2-e (SD+)

FIG_2-f (SD-)

FIG_2-g (VC)

FIG_2-h (haute tension) (T.P)

FIG_2-j (F.P)

2/2

0144272

**Office européen des brevets**

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 057 745  (R.D. ALBERT)<br>*  Abrégé;  colonne  3,  lignes 12-36;  colonne  5,  ligne  51  - colonne  6,  ligne  40;  figures 1,2,14 * | 1,3-5 | H 01 J   35/30<br>H 01 J   35/14<br>A 61 B    6/02 |
| A | GB-A-2 044 489  (EMI LTD.)<br>* Page 1, lignes 5-55, 91-116 * | 1,3,6 | |
| A | FR-A-2 384 415  (J. HAIMSON)<br>* Page 41, lignes 2-30 * | 1,3 | |
| A | FR-A-2 184 328  (R.D. ALBERT)<br>*  Page  7,  ligne  10  - page 9, ligne  4;  page 18, lignes 24-29; figure 1 * | 1-3 | |
| A | FR-A-2 441 267  (TOKYO SHIBAURA DENKI K.K.)<br>* Page 2, lignes 7-33 * | 1 | DOMAINES TECHNIQUES RECHERCHES (Int. Cl.4)<br><br>A 61 B    6/00<br>G 01 N   23/00<br>H 01 J   35/00<br>H 05 G    1/00 |

Le présent rapport de recherche a été établi pour toutes les revendications

| Lieu de la recherche | Date d'achèvement de la recherche | Examinateur |
|---|---|---|
| LA HAYE | 11-03-1985 | HORAK G.T. |